# EUROPÄISCHE PATENTSCHRIFT

(11) **EP 0 752 404 B1**
(45) Veröffentlichungstag und Bekanntmachung des Hinweises auf die Patenterteilung: **15.09.1999**
(21) Anmeldenummer: 96108301.1
(22) Anmeldetag: 24.05.1996
(51) Int. Cl.: C07C 29/124, C07C 33/22

(54) **Verfahren zur Herstellung von Benzylalkohol**
Process for the preparation of benzyl alcohol
Procédé pour la préparation de l'alcool benzylique

(30) Priorität: 06.06.1995 DE 19520612
(43) Veröffentlichungstag der Anmeldung: 08.01.1997
(73) Patentinhaber: BAYER AG, 51368 Leverkusen (DE)
(72) Erfinder: Buysch, Hans-Josef, Dr., 47809 Krefeld (DE); Hoffmann, Erhard-Günther, Dr., 40883 Ratingen (DE); Jansen, Ursula, Dr., 47799 Krefeld (DE); Ooms, Pieter, Dr., 47800 Krefeld (DE); Schenke, Bernd-Ulrich, 46236 Bottrop (DE)

(56) Entgegenhaltungen:
- DE-C- 484 662
- JUSTUS LIEBIGS ANNALEN DER CHEMIE, Bd. 195, 1879, LEIPZIG HEIDELBERG DE, Seiten 353-354, XP002024365 NIEDERIST: "Über die Einwirkung von Wasser auf die Haloidverbindungen der Alkoholradikale"

## Beschreibung

Die vorliegende Erfindung betrifft ein Verfahren zur Herstellung von Benzylalkohol durch Hydrolyse von Benzylchlorid mit Wasser im Molverhältnis 1 : 10 bis 1 : 70 bei 80 bis 180°C, wobei die Reaktanden ohne Zusatz eines wasserlöslichen Hilfsmittels intensiv durchmischt werden, die Reaktion bei unvollständigem Umsatz abgebrochen und die Reaktionsmischung aufgearbeitet wird.

Über die Hydrolyse von Benzylchlorid ist bereits sehr viel berichtet worden. Mehrere Publikationen beschäftigen sich mit diesem Problem zwecks Aufklärung des Reaktionsmechanismus und Erarbeitung analytischer Methoden. Dabei werden in der Regel homogene Mischungen aus Benzylchlorid, Wasser und einem wasserlöslichen Lösungsvermittler wie Alkohol, Aceton, Dioxan oder Essigsäure verwendet; siehe z.B. J. Chem. Soc. 1954, 1840 ff; Z. Naturf. 1946 (1) 580-4, J. Chem. Soc. 1957, 4747 ff; Tetrahedron 1957 (1) 129-144; Rec. 48, 227 ff (1929) und 49, 667 ff (1930). In verschiedenen Fällen wird die Hydrolyse auch in Gegenwart von Alkalihydroxiden, Alkalicarbonaten oder Erdalkalicarbonaten durchgeführt, wie z.B. in J.Am.Chem. Soc. 62, 2481 (1940), Rec. 53, 891 ff und 869 ff (1934), wobei letztere Veröffentlichungen zeigen, daß die Hydrolyse in Gegenwart von basischen Verbindungen beschleunigt wird. In allen diesen Arbeiten wird auf die Isolierung und Identifizierung der Hydrolyseprodukte kein Wert gelegt.

Es gibt jedoch auch Untersuchungen der Benzylchloridhydrolyse mit Wasser allein, die eine Beschreibung der Hydrolyseprodukte beinhalten. Nach Ann. 139, 307 f (1866) geht Benzylchlorid bei 190°C mit Wasser vor allem in Kohlenwasserstoffe und Dibenzylether über.

Unter milderen Bedingungen (Ann. 196, 353 (1879) bei 100 bis 110°C und vollständigem Umsatz erhielt man - allerdings in sehr starker Verdünnung - eine Ausbeute an Benzylalkohol von 76 % d.Th.; der Rest waren Hochsieder, die nicht charakterisiert wurden.

Die Erkenntnis, daß die Umsetzung in Gegenwart von Alkalien rascher und glatter verläuft, hat im technischen Bereich früh zur Bestrebung geführt, die während der Hydrolyse von Benzylchlorid entstehende Salzsäure zu binden. So wird im DRP 484 662 empfohlen, die Reaktion in Gegenwart von Calciumcarbonat durchzuführen, und in der US-PS 2 221 882 wird in Gegenwart zunächst einer Sodalösung und nachfolgend einer Natronlauge gearbeitet. Die Ausbeute liegt deutlich über 90 % d.Th.

Natriumcarbonat als Base hat sich offenbar bewährt und wurde in der Folgezeit beibehalten. Die weiteren Entwicklungen auf diesem Gebiet befassen sich mit der technischen Ausgestaltung dieser basischen Hydrolyse von Benzylchlorid als kontinuierliches Verfahren wie in DE-OS 2 101 810, EP-A 64 486 und DD-PS 161 067 beschrieben.

Nachteile der alkalischen Hydrolyse jedoch sind die zusätzliche Verwendung von Natriumcarbonat, die Bildung von Natriumchlorid und der Anfall von großen Mengen an wäßrigen Ablaugen, die entsorgt werden müssen.

Die Aufgabe der vorliegenden Erfindung bestand also darin, ein neues Verfahren zu entwickeln, das die Zugabe von Natriumcarbonat und damit Kochsalz- und Abwasserbelastung vermeidet.

Es wurde nun gefunden, daß man dies praktisch mit mindestens gleicher oder besserer Ausbeute an Benzylalkohol erreicht, wenn man bei Temperaturen zwischen 80°C und 180°C Benzylchlorid mit der 10- bis 70fachen Molmenge an Wasser unter intensiver Durchmischung reagieren läßt und unvollständig umsetzt.

Nach dem Stand der Kenntnisse war das Ergebnis keinesfalls zu erwarten: Die bisher bekannt gewordenen Ausbeuten waren mäßig und die Art der Nebenprodukte nicht bekannt. Die unter milden Bedingungen bei 100°C bis 110°C erhaltenen 76 % Benzylalkohol (Ann. 196, 353 (1879)) wurden bei einem Molverhältnis von Wasser : Benzylalkohol von 190 : 1, d.h. einem Gewichtsverhältnis von 27 : 1 gewonnen; dies bedeutet, daß die erhaltene Salzsäure einerseits zwar eine sehr niedrige Konzentration hatte und daher auch kaum die Nebenproduktbildung in Gang setzten konnte, andererseits aber auch keinen Gebrauchswert besaß.

Darüber hinaus war unter diesen Voraussetzungen eine technisch untragbar niedrige Raum-Zeit-Ausbeute zu erwarten.

Andererseits mußte bei einer Verminderung des Molverhältnisses unter 190 : 1 auf die anspruchsgemäßen Werte von 10 bis 70 : 1 mit einer deutlich vermehrten Nebenproduktbildung gerechnet werden, weil die Salzsäurekonzentration und damit ihre Aktivität um ein Mehrfaches steigt.

Demgegenüber ergibt das erfindungsgemäße Verfahren eine Salzsäure höherer Konzentration als Beiprodukt, das sich weiterverwenden läßt.

Geeignetes Ausgangsprodukt für das erfindungsgemäße Verfahren ist Benzylchlorid, wie es allgemein durch Seitenkettenchlorierung zugänglich ist.

Geeignet sind natürlich auch mit Halogen wie Chlor, Carboxyl, Cyan, Methoxy, Methyl und Nitro substituiertes Benzylchlorid. Bevorzugt ist jedoch unsubstituiertes Benzylchlorid.

Der Temperaturbereich, in dem die Umsetzung durchgeführt wird, beträgt 80 bis 180, bevorzugt 85 bis 170, besonders bevorzugt 90 bis 160°C.

Das Molverhältnis von Wasser zu Benzylchlorid beträgt bevorzugt 55 : 1 bis 15 : 1, besonders bevorzugt 50 : bis 20 : 1. 1

Der Umsatz an Benzylchlorid beträgt 35 bis 99 %, bevorzugt 40 bis 90 %, besonders bevorzugt 45 bis 85 %.

Bei der Durchführung der Reaktion oberhalb 100°C muß entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Die intensive Durchmischung der Reaktionspartner kann auf verschiedene dem Fachmann bekannte Weisen, etwa durch Rühren, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 568-570, Verlag Chemie, Weinheim 1988 näher beschrieben.

Die bei dem erfindungsgemäßen Verfahren benötigten Reaktionszeiten sind unerwartet kurz. Die genannten Umsätze von 35 bis 99 % an Benzylchlorid sind in wenigen Minuten bis einigen Stunden erreicht, etwa in 2 bis 240, bevorzugt 3 bis 180, besonders bevorzugt 4 bis 120 min.

Nach dem erfindungsgemäßen Verfahren erhält man eine Ausbeute an Benzylalkohol von > 90 bis ca. 96 %, die damit über der mit der alkalischen Hydrolyse erhältlichen von 85 % liegt, Ullmann's Encyclopedia of Industrial Chemistry, 5. Aufl., Vol.4, p.3, Weinheim. Nebenprodukt ist wie in den laufenden technischen Prozessen Dibenzylether.

Das erfindungsgemäße Verfahren entspricht also einer Benzylalkoholsynthese ohne Soda und ohne Abwasser; denn die erhaltene Salzsäure kann durch Absorption von HCl aus der Chlorierung aufkonzentriert und technisch weiterverwendet oder in einer Salzsäureelektrolyse wieder in Chlor umgewandelt werden.

Benzylalkohol eignet sich als Weichmacher und Lösungsmittel in der Lackindustrie, als Lösungsmittel für Riechstoffe und für spezielle Anwendungen in der Kautschuk- und Textilverarbeitung.

Die Prozentangaben der nachfolgenden Beispiele beziehen sich auf das Gewicht.

### Beispiele

### Beispiel 1

126,5 g (1 Mol) Benzylchlorid und 180 g (10 Mol) Wasser wurden in einem Kolben mit Strombrecher und Flügelrührer unter kräftigem Rühren (250 U/min) und unter Stickstoff rasch zum Sieden erhitzt. Nach 240 Min. Reaktionszeit wurde rasch abgekühlt, die organische Phase nach Zugabe von Toluol abgetrennt und gaschromatographisch analysiert. Man fand einen Umsatz von 48 g an Benzylchlorid und eine Ausbeute an Benzylalkohol von 37 g und ca. 3,6 g Dibenzylether, entsprechend einem Umsatz von 38 % und einer Ausbeute an Benzylalkohol von 91 %. Die entstandene Salzsäure war etwa 7,4 %ig.

### Beispiel 2

126,5 g (1 Mol) Benzylchlorid und 720 g (40 Mol) Wasser wurden in einem Kolben analog Beispiel 1 2 h unter Sieden gerührt und dann rasch abgekühlt. Nach Isolierung und Analyse der organischen Phase, wie in Beispiel 1, fand man einen Umsatz an Benzylchlorid von 76 % und eine umsatzbezogene Ausbeute an Benzylalkohol von 94 %. Nebenprodukt war wiederum Dibenzylether. Die entstandene Salzsäure enthielt etwa 3,8 % HCI.

### Beispiel 3

Beispiel 2 wurde wiederholt, aber mit 360 g (20 Mol) Wasser und nach 180 Min beendet. Der Umsatz betrug danach 64 % und die Ausbeute an Benzylalkohol 91 %.

### Beispiel 4

Beispiel 2 wurde wiederholt, aber mit 900 g (50 Mol) Wasser und nach 120 Min beendet. Der Umsatz betrug danach 92 % und die Ausbeute an Benzylalkohol 94 %.

### Beispiel 5

Beispiel 4 wurde wiederholt, aber bis zu einem Umsatz von 97 %, entsprechend einer Laufzeit von 3 h. Die Ausbeute an Benzylalkohol betrug 91 %.

### Beispiel 6

Beispiel 2 wurde wiederholt, aber mit 70 Mol Wasser und 2 h Laufzeit. Danach erhielt man einen Umsatz von 99 % und eine Benzylalkoholausbeute von 94 %.

### Beispiel 7

Beispiel 2 wurde wiederholt, aber bei 85°C und einer Laufzeit von 4,5 h. Man erhielt einen Umsatz von 52 % und eine Benzylalkoholausbeute von 96 %.

### Beispiel 8

Beispiel 2 wurde in einem Autoklaven bei 110°C wiederholt. Nach 50 min Laufzeit wurde ein Umsatz von 65 % und eine Ausbeute an Benzylalkohol von 95 % gefunden.

### Beispiel 9

Beispiel 2 wurde in einem Autoklaven bei 120°C wiederholt. Nach 15 Min. Laufzeit wurde ein Umsatz von 60 % und eine Ausbeute an Benzylalkohol von 96 % gefunden.

### Beispiel 10

Beispiel 2 wurde in einem Autoklaven bei 130°C wiederholt. Nach 10 Min. wurde ein Umsatz von 85 % und eine Benzylalkoholausbeute von 93 % erhalten.

### Beispiel 11

Beispiel 2 wurde in einem Autoklaven bei 140°C wiederholt. Nach 7 min Laufzeit wurde ein Umsatz von 85 % und eine Ausbeute an Benzylalkohol von 91 % gefunden.

### Beispiel 12

Beispiel 2 wurde in einem Autoklaven bei 150°C wiederholt, aber mit 540 g (30 mol) Wasser. Nach 4 Min. Laufzeit wurde ein Umsatz von 64 % und eine Ausbeute an Benzylalkohol von 94 % gefunden.

## Patentansprüche

1. Verfahren zur Herstellung von Benzylalkohol durch Hydrolyse von Benzylchlorid mit Wasser bei erhöhter Temperatur, dadurch gekennzeichnet, daß man bei einem Molverhältnis Benzylchlorid zu Wasser von 1 : 10 bis 1 : 70 und bei Temperaturen von 80 bis 180°C bis zu einem Umsatz an Benzylchlorid von 35 bis 99 % ohne Zusatz einer Base und ohne Zusatz eines wasserlöslichen organischen Lösungsmittels hydrolysiert.

## Claims

1. Process for the preparation of benzyl alcohol by the hydrolysis of benzyl chloride with water at elevated temperature, characterized in that the hydrolysis is performed at a molar ratio of benzyl chloride to water of from 1 : 10 to 1 : 70 and at temperatures of from 80 to 180°C until the conversion of benzyl chloride is from 35 to 99% without addition of a base and without addition of a water-soluble organic solvent.

## Revendications

1. Procédé de préparation de l'alcool benzylique par hydrolyse du chlorure de benzyle à l'eau pure et à température élevée, caractérisé en ce que l'on hydrolyse à un rapport molaire chlorure de benzyle/eau de 1:10 à 1:70 et à des températures de 80 à 180°C jusqu'à un taux de conversion du chlorure de benzyle de 35 à 99 %, sans adjonction d'une base et sans adjonction d'un solvant organique soluble dans l'eau.
